Europäisches Patentamt

**European Patent Office**

**Office européen des brevets**

(19)

(11) Veröffentlichungsnummer: **0 207 423**
A2

(12) **EUROPÄISCHE PATENTANMELDUNG**

(21) Anmeldenummer: **86108575.1**

(22) Anmeldetag: **24.06.86**

(51) Int. Cl.⁴: **C 12 N 1/00**
**C 12 N 1/08, C 12 P 21/00**

(30) Priorität: **29.06.85 DE 3523366**

(43) Veröffentlichungstag der Anmeldung:
**07.01.87 Patentblatt 87/2**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Deger, Hans-Matthias, Dr.**
**Am Rheingauer Weg 8**
**D-6238 Hofheim am Taunus(DE)**

(72) Erfinder: **Fricke, Ulrich**
**Am Flachsland 56**
**D-6233 Kelkheim (Taunus)(DE)**

(54) **Verfahren zur Gewinnung von mikrobiellem Eiweissisolat mit verbesserten Eigenschaften.**

(57) Mikrobielle Proteine können durch Reinigung der Fermentationsbrühe mit Hilfe eines ein- oder mehrstufigen Membrantrennverfahrens und anschließender Extraktion der Lipide und Nucleinsäuren von unerwünschten Geruchs- und Geschmacksstoffen sowie von Farbstoffen befreit werden.

EP 0 207 423 A2

Croydon Printing Company Ltd

— 1 —

## Verfahren zur Gewinnung von mikrobiellem Eiweißisolat mit verbesserten Eigenschaften

In der deutschen Offenlegunsschrift 2 137 038 wird beschrieben, daß Einzeller-Proteinmaterialien, insbesondere Hefen und Bakterien, durch Extraktion mit einer wäßrigen Lösung, die 60 bis 80 Vol-% eines niederen aliphatischen Alkohols enthält, von Substanzen mit unangenehmem Geschmack und Geruch befreit werden können.

Der Lipid- und Nucleinsäuregehalt in mikrobiellen Zellmassen kann ebenfalls durch Extraktion vermindert werden, wie aus der deutschen Patentschrift 2 633 666 hervorgeht. Hierzu werden die Zellmassen in einer ersten Stufe mit einer Extraktionsmischung aus Ammoniak und einem polaren Lösemittel aus der Reihe der niederen Alkanole, niederen Glykole und der Methyl- oder Ethylether eines niederen Glykols behandelt, wobei der Gesamtwassergehalt höchstens 30 Gew.-%, bezogen auf die Lösemittelmenge, und die Ammoniakkonzentration vorteilhaft 1 bis 10 Gew.-%, ebenfalls bezogen auf die Menge des Lösemittels, beträgt. Hierbei werden die Lipide und ein großer Teil der Geruchs- und Geschmacksstoffe extrahiert. In einer zweiten Stufe wird der Rückstand der Zellmassen ein- oder mehrstufig mit Wasser extrahiert, wodurch die Nukleinsäuren entfernt werden. Das so erhaltene Proteinisolat ist dann weitgehend geruch- und geschmacklos und kann für viele Anwendungszwecke, zum Teil auch in der menschlichen Ernährung, unmittelbar eingesetzt werden.

In der Praxis ist es jedoch nicht immer zu vermeiden, daß solchen Eiweißisolaten noch ein an Chemikalien erinnernder, salziger Geschmack und eine bräunlich-gelbe Färbung anhaftet. Dies stört insbesondere bei Anwendungsgebieten in der menschlichen Ernährung. Die Europäische Patentanmeldung 0 126 289 beschreibt ein Verfahren zur Entfernung von unerwünschten Geruchs- und Geschmacksstoffen aus mikrobiellen Proteinisolaten, indem man diese Materialien mit wäßrigen Lösungen behandelt, die ein Phosphat und/oder einen hydrophilen, niedermolekularen Aliphaten enthalten, die mindestens zwei funktionelle Gruppen aus der Reihe Hydroxy, Formyl, Keto und Carboxy enthält. Dieses Verfahren erreicht jedoch nur einen vergleichweise verbesserten Geruch und Geschmack und nicht geschmacksneutralität und ist außerdem zeit- und kostenaufwendig. Es ist daher wünschenswert, ein' farbloses, geschmacksneutrales Produkt, das vor allem auf dem Gebiet der menschlichen Ernährung universell einsetzbar ist, mit Hilfe eines einfachen Verfahrens herzustellen.

Es wurde nun gefunden, daß die Fermentationsbrühe, aus der das mikrobielle Proteinmaterial isoliert wird, durch ein- oder mehrstufige Membrantrennverfahren von Geruchs- und Geschmacksstoffen sowie von Farbstoffen befreit werden kann.

Die Erfindung betrifft somit ein Verfahren zur Herstellung von gereinigten und von Nucleinsäuren und Lipiden befreiten mikrobiellen Proteinisolaten, das dadurch gekennzeichnet ist, daß die Mikroorganismen enthaltende Fermentationsbrühe vor der Extraktion der Nucleinsäuren und/oder Lipide mit Hilfe eines Membrantrennverfahrens gereinigt wird.

In der folgenden Beschreibung werden bevorzugte Ausgestaltungen der Erfindung aufgeführt. In den Patentansprüchen wird die Erfindung definiert.
Als Ausgangsmaterial kommen grundsätzlich alle mikrobiellen Fermentationen in Betracht. Bevorzugt sind methylotrophe, vor allem obligat methylotrophe Mikroorganismen, insbesondere methanolverwertende Stämme, da Methanol eine billige, erdöl-unabhängige Kohlenstoffquelle darstellt. Methanolverwertende Mikroorganismen sind in großer Zahl bekannt und beispielsweise in den europäischen Patentanmeldungen 35 831 und 37 273, in der deutschen Auslegeschrift 2 161 164 und insbesondere in der deutschen Patentschrift 2 633 451 beschrieben. Diese Mikroorganismen wer-

- 3 -

den in an sich bekannter Weise kultiviert und die Kulturbrühe nach beendeter Fermentation erfindungsgemäß durch
ein Membrantrennverfahren, vorzugsweise Ultrafiltration,
gereinigt. Die Ultrafiltration in Platten-, Rohr-, Kapillarrohr-, Wickelmembran- und insbesondere Hohlfaserapparaten mit einer Ausschlußgrenze von 1000 bis 50 000 Dalton
ist besonders bevorzugt. Das die Membran nicht passierende
Retentat wird nach Trocknung bei 70 bis 90°C isoliert und
zur Gewinnung von Produkten für die menschliche Ernährung
von Nucleinsäuren und Lipiden befreit. Die Extraktion dieser Stoffe wird bevorzugt mit dem in der deutschen Patentschrift 2 633 666 genannten Verfahren durchgeführt, und
zwar mit einem Methanol/Ammoniak-Gemisch als Lipidlösemittel, wobei der Ammoniakgehalt vorzugsweise bei 1 bis 10
Gew.-%, bezogen auf die eingesetzte Lösemittelmenge, liegt
und anschließender Extraktion mit Wasser zur Entfernung der
Nucleinsäuren. Man erhält ein leicht gelbstichiges Proteinisolat mit sehr geringem Eigengeschmack.

Es ist vorteilhaft, die Biomasse durch Manipulation des pH-
Wertes bei erhöhter Temperatur zu flockulieren, um danach
die erfindungsgemäße Reinigung durch Ultrafiltration durchzuführen. Hierzu wird bei Temperaturen zwischen 50 und 60°C,
insbesondere bei 55°C gearbeitet. Der pH-Wert wird zuerst
mit einer Lauge, vorzugsweise Natronlauge, auf einen pH-Wert
von 7 bis 8 eingestellt und danach mit einer Säure, vorzugsweise Salzsäure, auf einen Wert von 4 bis 5 gesenkt.
Besonders vorteilhaft zum Abbau der Nucleinsäuren ist die
Behandlung der flockulierten Zellmasse mit 10.000 bis 40.000 U
einer Nuclease (1 g $\widehat{=}$ $10^6$ bis $10^7$ U) pro Liter Fermentationslösung mit ca. 10 - 15 g Biomasse. Die Enzymbehandlung wird
bei einer Temperatur von 50 bis 60°C und einem pH-Wert von 5 bis 6 durchgeführt. Bei der anschließenden Ultrafiltration werden die Abbauprodukte der
Nucleinsäure in das Permeat überführt. Durch die Behandlung erhält man
nach obengenannter Aufarbeitung ein geschmackneutrales, farbloses Proteinisolat.

Weitere detaillierte Ausgestaltungen werden im folgenden
anhand von Beispielen dargestellt. Die Prozentangaben beziehen sich auf das Gewicht.

Beispiel 1

Methylomonas clara ATCC 31 226 wurde gemäß deutscher Patentschrift 2 633 451 unter aeroben Bedingungen in einer Nährlösung gezüchtet, die Methanol als einzige Kohlenstoffquelle, Ammoniak als einzige Stickstoffquelle, Phosphat-, Eisen-, Magnesiumsalze und andere übliche Spurenelemente enthält. Man erhält eine Fermentationslösung mit etwa 14 g Biomasse pro Liter, in der die Zellmasse durch Einstellen des pH-Wertes mit Natronlauge auf 8 und danach mit Salzsäure auf 5 bei einer Temperatur von 55°C flokuliert wird. Aus 2 l dieser Fermentationslösung wird durch Sprühtrocknen bei 80°C ein Rohprotein gewonnen, das gemäß deutscher Patentschrift 2 633 666, Beispiel 1, von Lipiden durch Extraktion mit Methanol/Ammoniak im Verhältnis 10:1 und von Nucleinsäuren durch Extraktion mit Wasser befreit wird. Man erhält ein bräunlich-gelbes Isolat mit einem deutlich an Chemikalien erinnernden, salzigen Geschmack und einem Proteingehalt von 89,2%. Die Ausbeute beträgt etwa 23,8 g.

Beispiel 2

Man kultiviert Methylomonas clara ATCC 31 226 gemäß Beispiel 1. Zwei Liter der Kulturlösung mit 14 g Biomasse pro Liter werden über eine Ultrafiltrations-Kassette, die mit einer Zelluloseacetatmembran (Ausschlußgrenze 1 000 Dalton, Firma Millipore) beschickt ist, bei einem Vordruck von 3 bar und einem Flux von 25 l/m²h auf 200 ml aufkonzentriert. Das Konzentrat wird mit destilliertem Wasser auf 1 l aufgefüllt und der Ultrafiltrationsvorgang wiederholt bis erneut ein Endvolumen von 200 ml erreicht ist. Das Retentat wird durch Sprühtrocknen bei 80°C isoliert und gemäß Beispiel 1 von Lipiden und Nucleinsäuren befreit. Man erhält ein leicht gelbstichiges Isolat mit sehr geringem Eigengeschmack und einem Proteingehalt von 91,2 %. Die Ausbeute beträgt etwa 20,1 g.

Beispiel 3

Die Zellmasse aus 200 l einer Kulturlösung der Fermentation von Methylomonas clara ATCC 31 226 gemäß Beispiel 1 mit 12,8 g Biomasse pro Liter wird bei 55°C durch Einstellen des pH-Wertes mit Natronlauge auf 8 und danach mit Salzsäure auf 5 flockuliert. Die Suspension wird über ein Rohrmodul, das mit einer Zelluloseacetatmembran der Ausschlußgrenze 20 000 Dalton (Firma Kalle) belegt ist, durch Ultrafiltration bei einem Vordruck von 2,8 bar und einem Flux von 120 l/m²h auf ein Volumen von 40 Litern eingeengt. Nach Auffüllen auf das Ausgangsvolumen mit destilliertem Wasser wird der Ultrafiltrationsvorgang bis zu einem Endvolumen von 50 l wiederholt. Das Retentat wird anschließend durch Versprühen bei 80°C getrocknet, isoliert und gemäß Beispiel 1 von Lipiden und Nucleinsäuren befreit. Man erhält ein fast farbloses Pulver mit einem Proteingehalt von 91,5 %, das bei Verkostung als geschmacksneutral beurteilt wurde.

Beispiel 4

Man fermentiert Methylomonas clara ATCC 31 226 gemäß Beispiel 1 und flockuliert die Biomasse gemäß Beispiel 3. Die Suspension wird bei einer Temperatur von 55°C und einem pH-Wert von 5 nach Zugabe von 30.000 U 5'-Phosphodiesterase (Fa. Amano Nagoya, Japan) (1 g = 13 10$^6$ U) eine Stunde gerührt. Nach Abkühlen auf Raumtemperatur wurde die Lösung an einer Kassette, die mit einer Polysulfonmembran der Ausschlußgrenze 10 000 Dalton (Firma Millipore) belegt ist, bei einem Vordruck von 5 bar und einem Flux von 40 l/m²h auf 200 ml eingeengt. Der Ultrafiltrationsschritt wird nach Auffüllen auf jeweils 1 Liter mit destilliertem Wasser zweimal wiederholt. Das Retentat wird nach Sprühtrocknen bei maximal 80°C als Pulver isoliert und gemäß Beispiel 1 von Lipiden befreit. Man erhält ein farbloses, geschmacksneutrales Isolat mit einem Proteingehalt von 92,1% und einem Nucleinsäuregehalt von 1,8 %.

PATENTANSPRÜCHE:

1. Verfahren zur Herstellung von gereinigten und von Nucleinsäuren und Lipiden befreiten mikrobiellen Proteinisolaten, dadurch gekennzeichnet, daß die Mikroorganismen enthaltende Fermentationsbrühe vor der Extraktion der Nucleinsäuren und/oder Lipide mit Hilfe eines Membrantrennverfahrens gereinigt wird.

2. Verfahren nach Anspruch 1, dadurch gekennzeichnet, daß als Membrantrennverfahren die Ultrafiltration dient.

3. Verfahren nach Anspruch 2, dadurch gekennzeichnet, daß Ultrafiltrationsmembranen mit einer Ausschlußgrenze von 1 000 bis 50 000 Dalton eingesetzt werden.

4. Verfahren nach einem oder mehreren der Ansprüche 1 bis 3, dadurch gekennzeichnet, daß die Biomasse in der Fermentationsbrühe, vor der Reinigung durch ein Membrantrennverfahren, zur Flockulation gebracht wird.

5. Verfahren nach Anspruch 4, dadurch gekennzeichnet, daß die Flockulation der Biomasse in der Fermentationslösung bei einer Temperatur von 50 bis 60°C durch Einstellen des pH-Wertes auf 7 bis 8 und danach auf 4 bis 5 erreicht wird.

6. Verfahren nach einem oder mehreren der Ansprüche 1 bis 5, dadurch gekennzeichnet, daß die Fermentationsbrühe mit der flockulierten Biomasse, vor der Reinigung durch ein Membrantrennverfahren, mit einer Nuclease behandelt wird.

7. Verfahren nach Anspruch 6, dadurch gekennzeichnet, daß die Behandlung mit der Nuclease bei einer Temperatur von 50 bis 60°C und einem pH-Wert von 5 bis 6 erfolgt.

8. Verfahren nach Anspruch 6 oder 7, dadurch gekennzeichnet, daß Nuclease in Mengen von 10.000 bis 40.000 U/l eingesetzt wird.

9. Verfahren nach einem oder mehreren der Ansprüche 6 bis 8, dadurch gekennzeichnet, daß als Nuclease eine 5'-Phosphodiesterase eingesetzt wird.

10. Verfahren nach einem oder mehreren der Ansprüche 1 bis 9, dadurch gekennzeichnet, daß methylotrophe und/oder obligat methylotrophe und/oder methanolverwertende Mikroorganismen eingesetzt werden.